# EUROPÄISCHE PATENTSCHRIFT

(11) **EP 3 175 384 B1**
(45) Veröffentlichungstag und Bekanntmachung des Hinweises auf die Patenterteilung: **16.06.2021**
(21) Anmeldenummer: 15781901.2
(22) Anmeldetag: 12.10.2015
(51) Int. Cl.: G16H 40/63

(54) **VERFAHREN UND VORRICHTUNG ZUM LOGIN BEI MEDIZINISCHEN GERÄTEN**
METHOD AND APPARATUS FOR LOGGING INTO MEDICAL DEVICES
PROCÉDÉ ET DISPOSITIF DE CONNEXION À DES APPAREILS MÉDICINAUX

(30) Priorität: 14.10.2014 DE 102014220808
(43) Veröffentlichungstag der Anmeldung: 07.06.2017
(73) Patentinhaber: Siemens Healthcare GmbH, 91052 Erlangen (DE)
(72) Erfinder: ROMMEL, Michael, 91052 Erlangen (DE)
(86) Internationale Anmeldenummer: PCT/EP2015/073532
(87) Internationale Veröffentlichungsnummer: WO 2016/058976

(56) Entgegenhaltungen:
- EP-A1- 2 493 112
- EP-A1- 2 677 681
- WO-A1-2009/056897

## Beschreibung

### Gebiet der Erfindung

Die Erfindung betrifft ein Verfahren und eine Vorrichtung zum Login eines Servicetechnikers bei einem elektrischen Gerät, insbesondere bei einem medizinischen Gerät. Beispielsweise soll die Nutzung des elektrischen Geräts mittels einer Service Software nur nach Eingabe eines Login-Passwortes erlaubt sein.

### Hintergrund der Erfindung

Die Benutzung der Service Software eines elektrischen Geräts, insbesondere eines medizinischen Geräts, soll nur berechtigten Personen möglich sein. Bekannt ist, ein symmetrisches Schlüsselverfahrens zu nutzen, bei dem verschiedene Merkmale des medizinischen Geräts mit einem Geheimnis kombiniert und daraus ein License Key (= Lizenzschlüssel) erstellt werden. Das Geheimnis ist oftmals in einschlägigen Internetkreisen bekannt geworden oder die License Keys sind auf dem Schwarzmarkt verfügbar.

Verschiedene Schutzverfahren zur Verhinderung bzw. zum Erschweren eines Betrugs sind bekannt. Beim Public-/Private Key Verfahren auf Basis digitaler Zertifikate ist ein Handshake oder ein Challenge/Response Verfahren erforderlich. Die Etablierung einer Public Key Infrastruktur (PKI) erfordert einen regelmäßigen Zertifikatsupdate aufgrund auslaufender Gültigkeiten. Die Eingabe statischer Schlüsselinformationen verlangt eine große Schlüssellänge, um Brute-Force Attacken zu mäßigen. Große Schlüssellängen sind aber benutzerunfreundlich. Time-Based One-Time Passwörter haben die negative Eigenschaft, eine Zeitsynchronisation oder eine Online-Verbindung vorauszusetzen.

Aus der Druckschrift WO 2009/056897 A1 ist ein Verfahren zur Authentifizierung eines Benutzers im "Challenge-Response-Verfahren" bekannt, wobei die "Challenge" in einem Bild enthalten (beispielsweise in Form eines QR-Codes) an den Benutzer übertragen wird. Aus der Druckschrift EP 2 493 112 A1 sind Algorithmen zum geheimen Schlüsselaustausch bekannt.

### Zusammenfassung der Erfindung

Es ist Aufgabe der Erfindung, ein Verfahren und eine Vorrichtung anzugeben, die sicherstellen, dass eine Benutzung einer Service Software eines elektrischen Geräts nur berechtigten Personen möglich ist.

Gemäß der Erfindung wird die gestellte Aufgabe mit dem Verfahren und der Vorrichtung der unabhängigen Patentansprüche gelöst. Vorteilhafte Weiterbildungen sind in den abhängigen Ansprüchen angegeben.

Erfindungsgemäß bestehen das Verfahren und die zugehörige Vorrichtung darin, ein temporäres Login-Passwort und temporäres Schlüsselmaterial zu erzeugen, deren Gültigkeit auf den jeweiligen Login-Vorgang begrenzt ist. In Kombination mit einem Public-/Private Key Paar, das bei der Herstellung des Systems erzeugt wird und dessen Private Key auf einem mobilen Gerät, beispielsweise auf einem Smartphone eines Servicetechnikers, geschützt gespeichert ist, kann das temporäre Login-Passwort benutzerfreundlich kurz gehalten werden. Die Informationsübertragung zwischen dem elektrischen Gerät und dem mobilen Gerät erfolgt über einen QR Code und ist daher ebenfalls sehr nutzerfreundlich.

Für alle Public-/Private Schlüsselpaare können die bekannten Elliptic Curve Cryptoverfahren eingesetzt werden. Zur Erzeugung eines temporären gemeinsamen Geheimnisses, das zur Verschlüsselung des Login-Passworts benutzt wird, kann das etablierte Diffie-Hellman Schlüsselaustauschverfahren benutzt werden.

Die Erfindung beansprucht ein Verfahren zum Login eines Servicetechnikers bei einem elektrischen Gerät, wobei ein geheimer Schlüssel durch das elektrische Gerät als verschlüsseltes Login-Passwort erzeugt wird, der geheime Schlüssel auf einer Anzeigeeinheit des elektrischen Geräts als QR Code dargestellt wird, der QR Codes mit Hilfe eines mobilen Geräts optisch erfasst wird, das Login-Passwort durch das mobile Gerät aus dem geheimen Schlüssel des erfassten QR Codes entschlüsselt wird, das Login-Passwort auf einer Bildschirmeinheit des mobilen Geräts sichtbar gemacht wird, das Login-Passwort in das elektrische Gerät durch den Servicetechniker eingegeben wird, das eingegebene Login-Passwort mit dem erzeugten Login-Passwort durch das elektrische Gerät verglichen wird, der Login durch das elektrische Gerät freigegeben wird, wenn die beiden Login-Passwörter übereinstimmen.

Der Vorteil der Erfindung liegt in der Kombination der hohen Stärke der kryptografischen Sicherung mit der Benutzerfreundlichkeit des QR Codes und des einzugebenden relativ kurzen Login-Passworts. Die Erfindung bietet eine Möglichkeit, gerätespezifische Schlüssel mit großer Länge und benutzerfreundliche Login-Passwörter mittels des Einsatzes von Smartphone Technologien zu vereinen.

Erfindungsgemäß werden vor der Auslieferung des elektrischen Geräts in einer Servicezentrale ein erster Public Key und ein dazugehöriger erster Private Key erzeugt, wobei der erste Private Key in der Servicezentale gespeichert und der erste Public Key auf dem elektrischen Gerät installiert wird.

Erfindungsgemäß können die Verschlüsselung und die Entschlüsselung des Login-Passworts mit Hilfe eines gemeinsamen Geheimnisses erfolgen.

In einer weiteren Ausbildung kann das gemeinsame Geheimnis mittels des Elliptic-Curve-Diffie-Hellman-Schlüsselaustauschverfahrens errechnet bzw. erstellt werden.

Erfindungsgemäß kann das gemeinsame Geheimnis in dem mobilen Gerät mit Hilfe eines in dem elektrischen Gerät erzeugten zweiten Public Keys und des ersten Private Keys ermittelt werden, wobei vorteilhafterweise der erste Private Key in einem sicheren Speicherbereich des mobilen Geräts gespeichert ist bzw. wird.

In einer Weiterbildung kann der QR Code eine Kombination des geheimen Schlüssels, des zweiten Public Keys und einer Material-/Seriennummer des elektrischen Geräts enthalten.

In einer weiteren Ausführungsform kann das mobile Gerät mittels der Material-/Seriennummer den zu dem elektrischen Gerät gehörigen ersten Private Key zuordnen.

Des Weiteren kann das Login-Passwort zeitlich beschränkt gültig sein.

Die Erfindung beansprucht auch eine Vorrichtung zum Login eines Servicetechnikers bei einem elektrischen Gerät, wobei die Vorrichtung ein mobiles Gerät und das elektrische Gerät aufweist. Das elektrische Gerät und das mobile Gerät sind ausgebildet und programmiert, ein erfindungsgemäßes Verfahren auszuführen.

In einer Weiterbildung weist die Vorrichtung zusätzlich eine Servicezentrale auf, wobei die Servicezentrale, das elektrische Gerät und das mobile Gerät ausgebildet und programmiert sind, ein erfindungsgemäßes Verfahren auszuführen.

Die Servicezentrale, das elektrische Gerät und das mobile Gerät weisen zur Durchführung des Verfahrens u.a. jeweils eine elektronische Recheneinheit und eine Speichereinheit auf.

In einer weiteren Ausführungsform können das mobile Gerät ein Smartphone oder ein Tablet-Computer und das elektrische Gerät ein medizinisches Gerät sein.

Weitere Besonderheiten und Vorteile der Erfindung werden aus den nachfolgenden Erläuterungen eines Ausführungsbeispiels anhand von schematischen Zeichnungen ersichtlich.

Es zeigen:
- Fig. 1:: eine Vorrichtung zum Login und
- Fig. 2:: ein Ablaufdiagramm des Login-Verfahrens.

### Detaillierte Beschreibung eines Ausführungsbeispiels

Das erfindungsgemäße Verfahren und die erfindungsgemäße Vorrichtung werden beispielhaft für ein medizinisches Gerät, beispielsweise für einen Computertomographen oder einen Magentresonanztomographen, und ein Smartphone als mobiles Gerät beschrieben.

Der Schutz von Applikationen mit symmetrischen Schlüsseln erfordert eine lokale Speicherung eines Schlüssels auf dem medizinischen Gerät. Wenn aber viele medizinische Geräte denselben Schlüssel verwenden, steigt die Attraktivität für einen betrügerischen Angriff.

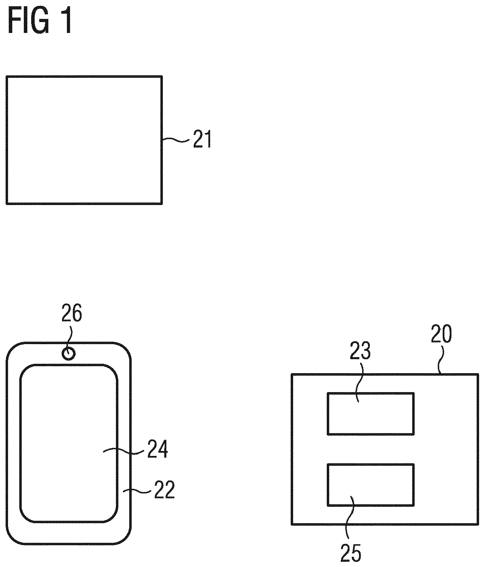

**Fig. 1** zeigt ein Blockschaltbild einer Vorrichtung zum sicheren Login auf einem medizinischen Gerät 20. Die Vorrichtung weist neben dem medizinischen Geräte 20 eine von dem medizinischen Gerät 20 entfernt gelegene Servicezentrale 21 und ein Smartphone 22 auf.

Die Vorrichtung dient dazu, ein Login-Verfahren für das medizinische Gerät 20 bereitzustellen, um einen Anwendungsschutz für das medizinische Gerät 20 zu realisieren, ohne dabei die Benutzerfreundlichkeit zu vernachlässigen. Das Login-Verfahren wird beispielsweise beim Service verwendet. Dabei müssen medizinische Geräte 20, die sich nach ihrer Auslieferung geografisch verteilt befinden, einer regelmäßigen Wartung unterzogen werden, wozu sich ein Servicetechniker an dem medizinischen Gerät 20 authentisieren muss.

Um die Speicherung eines privaten Schlüssels auf dem medizinischen Gerät 20 zu vermeiden, wird eine Public-Private Key Infrastruktur genutzt. Im angegebenen Verfahren kommt das etablierte Elliptic Curve Diffie-Hellman Schlüsselaustauschverfahren zum Einsatz.

Vor der Auslieferung des medizinischen Geräts 20 wird in der Servicezentrale 21 ein Elliptic Curve Public/Private Key Paar (erster Public Key PU1 und erster Private Key PR1) erzeugt. Der erste Private Key PR1 verbleibt in der Servicezentrale 21, der erste Public Key PU1 wird auf dem auszuliefernden medizinischen Gerät 20 installiert.

Die Kenntnis des ersten Private Key PR1 wird als Zugangsvoraussetzung zum medizinischen Gerät 20 akzeptiert. Die Länge des ersten Private Keys PR1 ist ausreichend groß, um einen guten Schutz gegen Faktorisierung zu gewährleisten. Der erste Private Key PK1 ist aber zu lang, um von einem Servicetechniker vor Ort von Hand eingegeben werden zu können.

Der Servicetechniker hat Zugriff auf das Smartphone 22 mit einer eingebauten Kamera 26 und mit einer servicespezifischen Login-Applikation. Zum Servicezeitpunkt ist eine direkte Online-Verbindung des Smartphones 22 zur Servicezentrale 21 in der Regel nicht erforderlich, jedoch muss in diesem Fall aber eine vorherige Synchronisation des Smartphones 22 mit einer zentralen Datenbank der Servicezentrale 21 erfolgen können.

Der funktionale Ablauf zur Erstellung eines Login-Passwortes LPW sieht wie folgt aus. Der Servicetechniker ruft die Login-Maske des medizinischen Geräts 20 auf. Das medizinische Gerät 20 stellt an einer Anzeigeeinheit 23 einen QR Code QRC dar und bietet ein Passwort-Eingabefeld an. Der Servicetechniker startet die Login-Applikation auf seinem Smartphone 22 und scannt mit der Kamera 26 den dargestellten QR Code QRC ein. Die Login-Applikation des Smartphones 22 zeigt ein, beispielsweise 12-stelliges, temporäres Login-Passwort LPW auf einer Bildschirmeinheit 24 des Smartphones 22 an. Der Servicetechniker gibt mit Hilfe einer Eingabeeinheit 25, zum Beispiel mit einer Tastatur, in der Login Maske des medizinischen Geräts 20 das temporäre Login-Passwort LPW ein. Der Login wird gewährt.

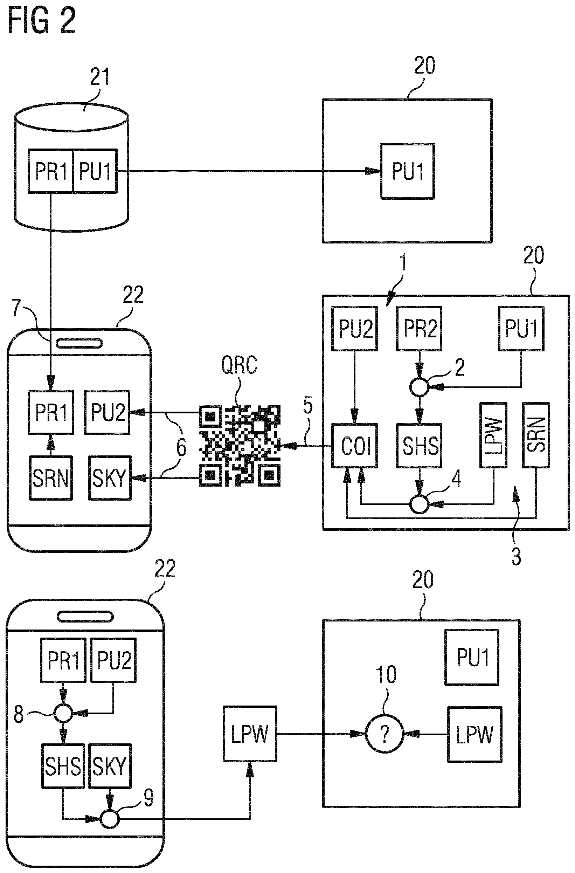

**Fig. 2** zeigt das Ablaufdiagramm des Logins mit einer Vorrichtung nach **Fig. 1****.** Beim Aufruf der Login-Maske an dem medizinischen Gerät 20 laufen folgende Schritte im Hintergrund ab. Im Schritt 1 erzeugt das medizinische Gerät 20 ein flüchtiges Public/Private Schlüsselpaar ("ephemeral", zweiter Public Key PU2 und zweiter Private Key PR2). Aus dem zweiten Private Key PR2 des flüchtigen Schlüsselpaars und dem ersten Public Key PU1 des medizinischen Geräts, der zum Auslieferungszeitpunkt erstellt wurde, wird im Schritt 2 ein gemeinsames Geheimnis SHS ("shared secret") erzeugt.

Im folgenden Schritt 3 generiert das medizinische Gerät 20 eine zufällige, beispielsweise 12-stellige, Ziffern/Buchstabenfolge (= Login-Passwort LPW), die später vom Servicetechniker als Eingabe erwartet wird. Intern wird ein Ablaufdatum festgelegt, das angibt, wie lange das medizinische Gerät 20 das temporäre Login-Passwort LPW als Login akzeptiert. Eine niedrigere Anzahl von Stellen kann optional ebenfalls genügen, wenn beispielsweise das Zeitfenster entsprechend reduziert wird.

Im Schritt 4 wird das Login-Passwort LPW mit einem symmetrischen Algorithmus und dem gemeinsamen Geheimnis SHS zum geheimer Schlüssel SKY ("Secret Key") verschlüsselt. Um die Sicherheit zu steigern, kann optional ein weiterer Zwischenschritt erfolgen, der den geheimen Schlüssel SKY hash'ed, bevor er als geheimer Schlüssel SKY verwendet wird, um sogenannte "weak bits" (= Bits, die mit begrenztem Aufwand vorhergesagt werden können) zu eliminieren (ECIES). Der geheime Schlüssel SKY wird Base64 kodiert und mit dem flüchtigen zweiten Public Key PU2 sowie der Material-/Seriennummer SRN des medizinischen Geräts 20 zur kombinierten Information COI kombiniert.

Anschließend wird im Schritt 5 die kombinierte Information COI der drei im Schritt 4 gewonnenen Informationen als QR Code QRC auf der Anzeigeeinheit 23 des medizinischen Geräts 20 dargestellt. Zu diesem Zeitpunkt sind öffentlich nur die beiden Public Keys PU1 und PU2 sowie der geheime Schlüssel SKY (also das verschlüsselte Login-Passwort LPW) bekannt. Alle anderen Informationen (Private Keys PR1 und PR2, gemeinsames Geheimnis SHS und Login-Passwort LPW) befinden sich nur im Hauptspeicher des medizinischen Geräts 20, in der Servicezentrale 21 oder im Smartphone 22.

Im nächsten Schritt 6 scannt der Servicetechniker mit dem Smartphone 22 den QR Code QRC von der Anzeigeeinheit 23 mit der Kamera 26 des Smartphones 22 ein, wodurch das Smartphone 22 über alle notwendigen Informationen zur Entschlüsselung des Login-Passworts LPW verfügt. Im folgenden Schritt 7 wird mit der Material-/Seriennummer SRN der passende erste Private Key PK1 des medizinischen Geräts 20 aus dem Smartphone 22 internen Speicherbereich geladen oder bei einer verfügbaren Mobilfunkverbindung direkt aus der Servicezentrale 21 über einen sicheren Übertragungskanal abgerufen.

Im Schritt 8 wird aus dem zweiten Public Key PU2 und dem nun verfügbaren ersten Private Key PK1 des medizinischen Geräts 20 das identische gemeinsame Geheimnis SHS errechnet. Mit dem gemeinsamen Geheimnis SHS dekodiert das Smartphone 22 im Schritt 9 den verschlüsselten geheimen Schlüssel SKY zum Login-Passwort LPW und stellt dieses auf der Bildschirmeinheit 24 des Smartphone 22 dar.

Nach Eingabe des Login-Passwortes LPW in das medizinische Gerät 20 vergleicht im Schritt 10 das medizinische Gerät 20 das eingegebene Login-Passwort LPW mit dem gespeicherten Login-Passwort LPW und entscheidet über den Login. Bei einer Übereinstimmung kann der Servicetechniker auf eine geschützte Applikation, beispielsweise auf eine Wartungssoftware, zugreifen.

Im Folgenden werden Angriffsszenarien und mögliche Gegenmaßnahmen beschrieben. Durch die Übertragung des ersten Private Keys PR1 des elektrischen Geräts 20 auf das mobile Gerät 22 des Servicetechnikers besteht ein erhöhtes Risiko, wenn das mobile Gerät 22 entwendet wird. Abhängig vom Schutz des mobilen Geräts 22 (zum Beispiel eine Entsperrcode oder Remote Wipe) kann der erste Private Key PR1 in die Hände eines Angreifers fallen. In diesem Fall wären dann aber nur diejenigen elektrischen Geräte 20 betroffen, deren erste Private Keys PR1 auf dem mobilen Gerät 22 gespeichert waren, nicht alle elektrischen Geräte 20.

Als Gegenmaßnahme kann die maximale Anzahl der auf dem mobilen Gerät 22 gespeicherten ersten Private Keys PR1 beschränkt werden. Kann eine Online Verbindung jederzeit vorausgesetzt werden, kann auf eine Vorab-Synchronisation des mobilen Geräts 22 vor einem Einsatz des Servicetechnikers verzichtet werden oder die Maximalzahl der gespeicherten ersten Private Keys PR1 sehr niedrig gesetzt werden. Optional kann die Applikation zwanghaft ein Startpasswort verlangen.

Die Gefahr von Man-in-the-Middle Attacken ist aufgrund des statischen Factory Keys (in der Servicezentrale generiert) und der Sichtverbindung beim Austausch von Nachrichten zwischen dem elektrischen Gerät 20 und dem mobilen Gerät 22 nicht gegeben.

Jederzeit kann ein Angreifer im Feld die Login Seite auf dem elektrischen Gerät 20 aufrufen und damit den QR Code QRC scannen. Brute Force Attacken auf die verwendeten kryptographischen Verfahren sind bislang bei Diffie-Hellman Schlüsselaustauschverfahren mit Schlüsseln in ausreichender Länge extrem aufwändig. Als Gegenmaßnahme werden ausreichend große Schlüssellängen verwendet, deren öffentlich gemachter geheimer Schlüssel SKY in base64 Kodierung als QR Code QRC noch vertretbar dekodierbar ist.

Das Passwortfeld könnte außerdem mittels Brute Force ausgetestet werden. Diese Methode wird bei Passwortlängen von 6-12 Zeichen schneller zum Ziel führen, als ein Angriff auf das ECDHE Verfahren. Als Gegenmaßnahme sollten maximal lange Login-Passworten LPW verwendet werden, die für eine manuelle Eingabe durch den Servicetechniker noch zumutbar sind. Weiterhin kann eine Sperrung des Zugangs nach einer bestimmten Anzahl von Falschversuchen in die zu schützende Applikation eingebaut werden. Dieser Schutz kann entweder zeitlich begrenzt (z.B. mit einer progressiven Sperrdauer) oder so eingebaut werden, dass nur eine Remote Administration die Sperre aufheben kann.

Da sowohl das temporäre Schlüsselpaar als auch das Login-Password LPW jedes Mal neu per Zufall erzeugt werden, hängt die Sicherheit in gewissem Masse von der Qualität des Zufallszahlengenerators ab. Kann ein Angreifer das eingegebene Passwort mitschneiden, könnte er dieses Passwort wiederverwenden. Als Gegenmaßnahme werden erprobte Algorithmen zur Erzeugung von Pseudozufallszahlen verwendet. Außerdem akzeptiert das elektrische Gerät 20 ein generiertes Login-Passwort LPW nur für einen begrenzten Zeitraum. Und schließlich kann nach einem erfolgreichen Login sofort das letzte erzeugte Login-Passwort LPW ungültig werden.

Die in heutigen Browsern unterstützten zwei Elliptischen Kurven (prime256v1 und secp384r1) sind bei kryptografischen Experten hinsichtlich ihrer kryptografischen Stärke in Diskussion. Von Koblitz Kurven ist die kryptografische Stärke nicht genau bekannt. Im Hinblick auf die Einflussnahme zentraler amerikanischer Institutionen (z.B. NIST) auf die Standardisierungsgremien und der Tatsache, dass beide oben genannten Kurven diejenigen sind, die die NSA Suite B security requirements erfüllen, befürworten manche Experten den Einsatz neuartiger Kurven, die nicht mit Beteiligung von NIST entwickelt wurden, zum Beispiel die curve 25519. Da die gesamte Login-Applikation in den Händen eines Herstellers liegt, kann sich der Einsatz derartiger Kurven lohnen.

Obwohl die Erfindung im Detail durch das Ausführungsbeispiel näher illustriert und beschrieben wurde, ist die Erfindung durch die offenbarten Beispiele nicht eingeschränkt und andere Variationen können vom Fachmann daraus abgeleitet werden, ohne den Schutzumfang der Erfindung zu verlassen, wie er durch die beigefügten Ansprüche definiert ist. Insbesondere ist die Erfindung nicht auf medizinische Geräte beschränkt, sondern kann für alle Geräte, die eine Rechen- und Speichereinheit besitzen, verwendet werden.

### Bezugszeichenliste

- 1: Erzeugung eines Schlüsselpaars PU1 und PR1
- 2: Erzeugung eines gemeinsamen Geheimnisse SHS
- 3: Generierung eines Login-Passworts LPW
- 4: Verschlüsselung des Login-Passworts LPW
- 5: Erzeugung und Darstellung des QR Codes QRC
- 6: Einscannen und Decodieren des QR Codes QRC
- 7: Laden des ersten Private Keys PR1
- 8: Errechnung des gemeinsamen Geheimnisses SHS
- 9: Dekodierung des geheimen Schlüssels SKY
- 10: Vergleich der Login-Passwörter LPW
- 20: Medizinisches Gerät
- 21: Servicezentrale
- 22: Smartphone
- 23: Anzeigeeinheit des medizinischen Geräts 20
- 24: Bildschirmeinheit des Smartphones 22
- 25: Eingabeeinheit des medizinischen Geräts 20
- 26: Kamera des Smartphones 22

- COI: kombinierte Information
- LPW: Login-Passwort
- PR1: erster Private Key
- PR2: zweiter Private Key
- PU1: erster Public Key
- PU2: zweiter Public Key
- QRC: QR Code
- SHS: gemeinsames Geheimnis
- SKY: geheimer Schlüssel
- SRN: Material-/Seriennummer des medizinischen Geräts 20

## Patentansprüche

1. Verfahren zum Login eines Servicetechnikers bei einem elektrischen Gerät (20) mit den Schritten:
- Erzeugung (3, 4) eines geheimen Schlüssels durch das elektrische Gerät (20) als verschlüsseltes Login-Passwort,
- Darstellung (5) des geheimen Schlüssels auf einer Anzeigeeinheit (23) des elektrischen Geräts (20) als QR Code,
- optische Erfassung (6) des QR Codes mit Hilfe eines mobilen Geräts (22),
- Entschlüsselung (9) des Login-Passworts durch das mobile Gerät (22) aus dem geheimen Schlüssel des erfassten QR Codes,
- Darstellung des Login-Passworts auf einer Bildschirmeinheit (24) des mobilen Geräts (22),
- Eingabe des Login-Passworts in das elektrische Gerät (20) durch den Servicetechniker,
- Vergleich (10) des eingegebene Login-Passworts mit dem erzeugten Login-Passwort durch das elektrische Gerät (20) und
- Freigabe des Logins durch das elektrische Gerät (20), wenn die beiden Login-Passwörter übereinstimmen,
wobei die Verschlüsselung (3, 4) und die Entschlüsselung (9) des Login-Passworts mit Hilfe eines gemeinsamen Geheimnisses erfolgen,
**dadurch gekennzeichnet, dass**
vor der Auslieferung des elektrischen Geräts (20) in einer Servicezentrale (21) ein erster Public Key und ein dazugehöriger erster Private Key erzeugt werden, wobei der erste Private Key in der Servicezentale (21) gespeichert und der erste Public Key auf dem elektrischen Gerät (20) installiert wird, wobei das gemeinsame Geheimnis in dem mobilen Gerät (22) mit Hilfe eines in dem elektrischen Gerät (20) erzeugten zweiten Public Keys und des ersten Private Keys ermittelt wird und wobei das gemeinsame Geheimnis im elektrischen Gerät (20) aus einem zweiten Private Key, welcher im elektrischen Gerät (20) erzeugt wird, und dem auf dem elektrischen Gerät (20) installierten ersten Public Key erstellt wird.

2. Verfahren nach Anspruch 1, wobei das gemeinsame Geheimnis mittels des Elliptic-Curve-Diffie-Hellman-Schlüsselaustauschverfahrens erstellt wird.

3. Verfahren nach Anspruch 1 oder 2, wobei der erste Private Key in einem sicheren Speicherbereich des mobilen Geräts (22) gespeichert ist.

4. Verfahren nach einem der vorstehenden Ansprüche wobei der QR Code eine Kombination des geheimen Schlüssels, des zweiten Public Keys und einer Material-/Seriennummer des elektrischen Geräts (20) enthält.

5. Verfahren nach Anspruch 4, wobei das mobile Gerät (22) mittels der Material-/Seriennummer den zu dem elektrischen Gerät (20) gehörigen ersten Private Key zuordnet.

6. Verfahren nach einem der vorhergehenden Ansprüche wobei das Login-Passwort zeitlich beschränkt gültig ist.

7. Vorrichtung aufweisend ein mobiles Gerät (22) und ein elektrisches Gerät (20) zum Login eines Servicetechnikers bei dem elektrischen Gerät (20), wobei das elektrische Gerät (20) und das mobile Gerät (22) ausgebildet und programmiert sind, ein Verfahren nach Anspruch 1 auszuführen.

8. Vorrichtung nach Anspruch 7, umfassend
- eine Servicezentrale (21),
- wobei die Servicezentrale (21), das elektrische Gerät (20) und das mobile Gerät (22) ausgebildet und programmiert sind, ein Verfahren nach einem der Ansprüche 2 bis 6 auszuführen.

9. Vorrichtung nach Anspruch 7 oder 8, wobei das mobile Gerät (22) ein Smartphone oder ein Tablet-Computer und das elektrische Gerät (20) ein medizinisches Gerät sind.

## Claims

1. Method for logging a service technician into an electrical device (20), comprising the steps of:
- generation (3, 4) of a secret key by the electrical device (20) as an encrypted log-in password,
- representation (5) of the secret key on a display unit (23) of the electrical device (20) as a QR code,
- optical detection (6) of the QR code with the aid of a mobile device (22),
- decryption (9) of the log-in password by the mobile device (22) from the secret key of the detected QR code,
- representation of the log-in password on a screen unit (24) of the mobile device (22),
- input of the log-in password into the electrical device (20) by the service technician,
- comparison (10) of the log-in password input with the generated log-in password by the electrical device (20), and
- release of the log-in by the electrical device (20) when the two log-in passwords match,
wherein the encryption (3, 4) and the decryption (9) of the log-in password take place with the aid of a shared secret,
**characterized in that**
before the delivery of the electrical device (20) in a service center (21), a first public key and an associated first private key are generated, the first private key being stored in the service center (21) and the first public key being installed on the electrical device (20),
wherein the shared secret is determined in the mobile device (22) with the aid of a second public key generated in the electrical device (20) and the first private key and
wherein the shared secret is generated in the electrical device (20) from a second private key generated in the electrical device (20) and the first public key installed on the electrical device (20).

2. Method according to Claim 1, wherein the shared secret is generated by means of the elliptic curve Diffie-Hellman key exchange method.

3. Method according to Claim 1 or 2, wherein the first private key is stored in a secure storage area of the mobile device (22).

4. Method according to one of the preceding claims, wherein the QR code contains a combination of the secret key, the second public key and a material/serial number of the electrical device (20).

5. Method according to Claim 4, wherein the mobile device (22) allocates the first private key belonging to the electrical device (20) by means of the material/serial number.

6. Method according to one of the preceding claims, wherein the log-in password is valid in a manner restricted in time.

7. Apparatus having a mobile device (22) and an electrical device (20) for logging a service technician into the electrical device (20), wherein the electrical device (20) and the mobile device (22) are designed and programmed to carry out a method according to Claim 1.

8. Apparatus according to Claim 7, comprising
- a service center (21),
- wherein the service center (21), the electrical device (20) and the mobile device (22) are designed and programmed to carry out a method according to one of Claims 2 to 6.

9. Apparatus according to Claim 7 or 8, wherein the mobile device (22) is a smartphone or a tablet computer and the electrical device (20) is a medical device.

## Revendications

1. Procédé pour le login d'un technicien de service dans un appareil (20) électrique, comprenant les stades :
- production (3,4) d'une clé privée par l'appareil (20) électrique comme mot de passe de login chiffré,
- représentation (5) de la clé privée sur une unité (23) d'affichage de l'appareil (20) électrique comme code QR,
- détection (6) optique du code QR à l'aide d'un appareil (22) mobile,
- déchiffrement (9) du mot de passe de login par l'appareil (22) mobile à partir de la clé privée du code QR détecté,
- représentation du mot de passe de login sur une unité (24) d'écran de l'appareil (22) mobile,
- entrée du mot de passe de login dans l'appareil (20) électrique par le technicien de service,
- comparaison (10) par l'appareil (20) électrique du mot de passe de login entré au mot de passe de login produit, et
- validation du login par l'appareil (20) électrique, si les deux mots de passe de login coïncident,
dans lequel le chiffrement (3, 4) et le déchiffrement (9) du mot de passe de login s'effectuent à l'aide d'un secret commun,
**caractérisé en ce que**
avant la livraison de l'appareil (20) électrique, on produit dans un central (21) de service une première public key et une première private key s'y rapportant, dans lequel on met la première private key en mémoire dans le central (21) de service et on installe la première public key sur l'appareil (20) électrique, dans lequel on détermine le secret commun dans l'appareil (22) mobile à l'aide d'une deuxième public key,produite dans l'appareil (20) électrique, et de la première private key et dans lequel on établit le secret commun dans l'appareil (20) électrique à partir d'une deuxième private key, qui est produite dans l'appareil (20) électrique, et de la première public key installée sur l'appareil (20) électrique.

2. Procédé suivant la revendication 1, dans lequel on établit le secret commun au moyen d'un procédé d'échange de clé Elliptic-Curve-Diffie-Hellman.

3. Procédé suivant la revendication 1 ou 2, dans lequel on met en mémoire la première private key dans une partie de mémoire sécurisée de l'appareil (22) mobile.

4. Procédé suivant l'une des revendications précédentes, dans lequel le code QR contient une combinaison de la clé privée, de la deuxième public key et d'un numéro de matériel/série de l'appareil (20) électrique.

5. Procédé suivant la revendication 4, dans lequel l'appareil (22) mobile associe, au moyen du numéro de matériel/série, la première private key se rapportant à l'appareil (20) électrique.

6. Procédé suivant l'une des revendications précédentes, dans lequel le mot de passe de login est valable d'une manière limitée dans le temps.

7. Installation comportant un appareil (22) mobile et un appareil (20) électrique pour le login d'un technicien de service dans l'appareil (20) électrique, dans lequel l'appareil (20) électrique et l'appareil (22) mobile sont constitués et programmés pour effectuer un procédé suivant la revendication 1.

8. Installation suivant la revendication 7, comprenant
- un central (21) de service,
- dans laquelle le central (21) de service, l'appareil (20) électrique et l'appareil (22) mobile sont constitués et programmés pour effectuer un procédé suivant l'une des revendications 2 à 6.

9. Installation suivant la revendication 7 ou 8, dans laquelle l'appareil (22) mobile est un téléphone intelligent ou un ordinateur à tablette et l'appareil (20) électrique est un appareil médical.
